(19) Europäisches Patentamt
European Patent Office
Office européen des brevets

(11) Veröffentlichungsnummer: **0 010 691**
**B1**

(12) # EUROPÄISCHE PATENTSCHRIFT

(45) Veröffentlichungstag der Patentschrift: **22.12.82**

(51) Int. Cl.³: **C 07 D 249/08**, A 01 N 43/64

(21) Anmeldenummer: **79104019.9**

(22) Anmeldetag: **18.10.79**

(54) **Neue 1,2,4-Triazolderivate Verfahren zu ihrer Herstellung, ihre Verwendung zur Herstellung von Pflanzenschutzmitteln, Verfahren zur Herstellung von Fungiziden und Verfahren zur Bekämpfung von Fungi sowie fungizide Mittel.**

(30) Priorität: **23.10.78 DE 2846038**

(43) Veröffentlichungstag der Anmeldung: **14.05.80 Patentblatt 80/10**

(45) Bekanntmachung des Hinweises auf die Patenterteilung: **22.12.82 Patentblatt 82/51**

(84) Benannte Vertragsstaaten: **AT BE CH DE FR GB IT LU NL SE**

(56) Entgegenhaltungen:
**CH-A-587 012**
**DE-A-2 201 063**
**DE-A-2 431 073**
**DE-A-2 635 663**
**DE-A-2 705 678**
**DE-A-2 812 287**
**US-A-3 972 891**
**US-A-4 013 677**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Stubenrauch, Gerd, Dr. Dipl.-Chem., Rubensstrasse 36, D-6700 Ludwigshafen (DE)**
Erfinder: **Ammermann, Eberhard, Dr. Dipl.-Chem., Sachsenstrasse 3, D-6700 Ludwigshafen (DE)**
Erfinder: **Hamprecht, Gerhard, Dr. Dipl.-Chem., Rote-Turm-Strasse 28, D-6940 Weinheim (DE)**
Erfinder: **Pommer, Ernst-Heinrich, Dr., Berliner Platz 7, D-6703 Limburgerhof (DE)**

EP 0 010 691 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue 1,2,4-Triazolderivate, Verfahren zu ihrer Herstellung, ihre Verwendung zur Herstellung von Pflanzenschutzmitteln, Verfahren zur Herstellung von Fungiziden und Verfahren zur Bekämpfung von Fungi sowie fungizides Mittel

Die vorliegende Erfindung betrifft neue 1,2,4-Triazolverbindungen, Verfahren zur Herstellung dieser Verbindungen, deren Verwendung zur Herstellung von Pflanzenschutzmitteln sowie Fungizide, welche diese Verbindungen enthalten.

Es ist bereits bekannt, dass man O,N-Acetale von α-Ketocarbonsäuren erhält, wenn man

a) Dichloressigsäure mit Alkoholaten in symmetrische Glyoxylsäure-O,O-acetale überführt (vgl. Houben-Weyl, «Methoden der organischen Chemie», Band 6/3, Seiten 239/240, G.Thieme-Verlag, Stuttgart, 1965), anschliessend mit Säurehalogeniden zu 2-Halogen-2-alkoxyessigsäurederivaten umsetzt (vgl. Chem. Ber. 99 (1966) 3260 ff) und diese mit Aminen in die gewünschten Glyoxylsäure-O,N-acetale überführt (vgl. Liebigs Ann. Chem. 702 (1967) 68 ff),

b) Dichloressigsäureester mit Aminen zunächst zu symmetrischen N,N-Acetalen des entsprechenden Glyoxylsäureamids umsetzt (vgl. Liebigs Ann. Chem. 702 (1967) 70), diese in die symmetrischen N,N-Acylale überführt und letztere mit Alkoholen zu N-acylierten N,O-Acetalen des entsprechenden Glyoxylsäureamids reagieren lässt (vgl. J. prakt. Chem. 311 (1969) 497 ff),

c) Glyoxylsäure mit Säureamiden zu 2-N-Acylamino-2-hydroxyessigsäuren umsetzt und diese mit alkoholischer Schwefelsäure in N-acylierte N,O-Acetale des entsprechenden Glyoxylsäureesters überführt (vgl. Tetrahedron 31 (1975) 863 ff) oder

d) Aminosäuren zu Oxazolonen-5 dehydratisiert, diese zu 4-Halogenoxazolonen-5 halogeniert und durch Ringspaltung und Alkoholyse in die gewünschten N-acylierten N,O-Acetale der entsprechenden α-Ketocarbonsäureester überführt (vgl. Mem. Bull. Soc. Chim. France (1959) 530 ff).

Diese Verfahren haben zunächst den Nachteil, dass zur Herstellung der Endstufe stets mehrere Reaktionsschritte nötig sind, so dass die Endausbeuten, bezogen auf die Ausgangsstoffe, sehr klein sind. Weiterhin ist der Anwendungsbereich dieser Methoden eng begrenzt, so dass beispielsweise O-Aryl-α-ketocarbonsäure-O,N-acetale so nicht hergestellt werden können.

Weiter sind aus der DE-OS 2 705 678, DE-OS 2 635 663, US-PS 3 972 891, US-PS 4 013 677, DE-OS 2 431 073, DE-OS 2 812 287, DE-OS 2 201 063 und der CH-PS 587 012 eine Vielzahl von Diazol- und insbesondere Triazol-Verbindungen bekannt, deren fungizide Wirkung jedoch unbefriedigend ist.

Das nachstehend beschriebene Verfahren der vorliegenden Anmeldung eröffnet nun einen überraschend einfachen Zugang zu neuen α-Ketocarbonsäure-O,N-acetalen. Die erfindungsgemässen Stoffe zeigen selbst gute fungizide Wirkungen und stellen darüber hinaus wertvolle Zwischenprodukte zur Darstellung von Pflanzenschutzmitteln (vgl. EP-OS 79 104 402) dar.

Gegenstand der Erfindung sind 1,2,4-Triazolderivate der Formel I

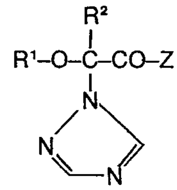 (I)

worin

$R^1$ einen durch 1 bis 3 Halogenatome, $C_{1-4}$-Alkylgruppen, eine Cyclohexyl- oder Phenylgruppe substituierten Phenylrest,

$R^2$ ein Wasserstoffatom, eine $C_{1-6}$-Alkyl- oder Phenylgruppe und

Z eine Hydroxylgruppe oder einen Rest $-O^{\ominus}M^{\oplus}$, worin $M^{\oplus}$ für ein Äquivalent eines Metallkations oder ein gegebenenfalls substituiertes Ammoniumion steht,

bedeutet.

Die Verbindungen der Formel I besitzen ein oder gegebenenfalls mehrere chirale Zentren; reine Isomere können aus den bei der Synthese anfallenden Gemischen nach den in der Technik bekannten Verfahren erhalten werden.

Verbindungen der Formel I können auch in zwitterionischer Form vorliegen und werden auch in dieser Form beansprucht.

Gegenstand der Erfindung ist weiter ein Verfahren zur Herstellung der Verbindungen der allgemeinen Formel I, welches dadurch gekennzeichnet ist, dass man Verbindungen der allgemeinen Formel II

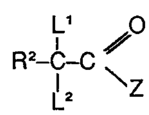 (II),

worin $R^2$ und Z die oben genannten Bedeutungen haben und $L^1$ und $L^2$ für nucleophil verdrängbare Abgangsgruppen stehen, mit einer Hydroxyverbindung der allgemeinen Formel III

$$R^1\text{–}OH \quad \text{(III)},$$

worin $R^1$ die oben genannte Bedeutung besitzt, und 1,2,4-Triazol, gegebenenfalls in Gegenwart eines Lösungs- oder Verdünnungsmittels und/oder eines Säurefängers und gegebenenfalls in Gegenwart eines Reaktionsbeschleunigers bei Temperaturen zwischen 0 und 180 °C umsetzt.

Als nucleophil verdrängbare Abgangsgruppen eignen sich besonders Halogen, Hydrogensulfat, Halogensulfonat, gegebenenfalls substituierte Arylsulfonyloxy-, Alkylsulfonyloxy-, Alkylsulfat-, Oxonium-, Sulfonium- oder Ammonium-Reste.

Anstelle der freien Wasserstoffverbindungen der Formel III und des 1,2,4-Triazols können auch entsprechende Salze, die nach bekannten Methoden in einer Vorreaktion oder gegebenenfalls durch Zugabe der äquivalenten Menge einer organischen oder anorganischen Base in situ erzeugt werden, eingesetzt werden, beispielsweise Alkali-, Erdalkali- oder gegebenenfalls substituierte Ammoniumsalze.

Verwendet man Phenol, 1,2,4-Triazol und Dichloressigsäure als Ausgangsstoffe, kann der Reaktionsablauf des erfindungsgemässen Verfahrens durch das folgende Formelschema wiedergegeben werden:

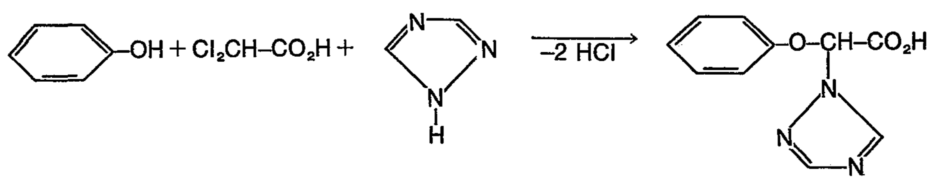


Zu den bevorzugten Lösungs- oder Verdünnungsmitteln gehören polare Lösungsmittel wie Wasser; Formamide, wie Dimethylformamid, Formamid, Dimethylacetamid; Nitrile, wie Acetonitril, Benzonitril, Butyronitril; Sulfoxide, wie Dimethylsulfoxid; Phosphorsäureamide, wie Hexamethylphosphorsäuretriamid; Ketone, wie Aceton, Ethylmethylketon, Cyclohexanon, Acetophenon; Ether, wie Tetrahydrofuran, Anisol, Dimethoxyethan, n-Butylethylether, Dioxan; Nitroalkane, wie Nitromethan; Nitrobenzol; Alkohole, wie Methanol, Ethanol, Isopropanol, n-Butanol, 3-Methylbutanol; Harnstoffe, wie Tetramethylharnstoff; Etheralkohole, wie Ethylenglykolmonomethylether, Sulfone, wie Sulfolan; Ester, wie Essigsäureethylester, Propionsäuremethylester, Ameisensäuremethylester. Daneben kommen jedoch auch in Frage: Halogenkohlenwasserstoffe, insbesondere Chlorkohlenwasserstoffe, beispielsweise Methylenchlorid, Chloroform, 1,2-Dichlorethan, 1,1,2,2- oder 1,1,1,2-Tetrachlorethan, Dichlorpropan, Trichlorethylen, Chlorbenzol, o,m,p-Dichlorbenzol, Fluorbenzol, o-, m- oder p-Chlortoluol, Dichlornaphthalin, Tetrachlorkohlenstoff; aliphatische oder cycloaliphatische Kohlenwasserstoffe, wie Heptan, Pinan, o,m,p-Cymol, Benzinfraktionen innerhalb eines Siedepunktsintervalles von 70 bis 190 °C, Cyclohexan, Methylcyclohexan, Dekalin, Petrolether, Ligroin, 2,2,4-Trimethylpentan, Octan; aromatische Kohlenwasserstoffe, wie Benzol, Toluol, o,m,p-Xylol, Tetralin, und entsprechende Gemische. Zweckmässig verwendet man das Lösungsmittel in einer Menge von 100 bis 2000 Gew.-%, vorzugsweise 100 bis 1000 Gew.-%, bezogen auf die Ausgangsstoffe der Formeln III bzw. IV.

Das erfindungsgemässe Verfahren wird vorzugsweise in Gegenwart einer organischen oder anorganischen Base vorgenommen. Insbesondere werden basische Hydroxyverbindungen, basische Oxide, tertiäre Amine, Alkoholate, Carbonate oder Hydride verwendet, beispielsweise Kaliumhydroxid, Natriumhydroxid, Kaliumcarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Zinkhydroxid, Zinkoxid, Zinkcarbonat, Zinkbicarbonat, Natriummethylat, Magnesiumethylat, Kaliumethylat, Natriumpropylat, Aluminiumisopropylat, Natriumbutylat, Lithiummethylat, Kaliumcyclohexanolat, Natriumisopropylat, Kalium-tert-butylat, Trimethylamin, Triethylamin, Tripropylamin, Triisopropylamin, Tributylamin, Triisobutylamin, Tri-sec-butylamin, Tri-tert-butylamin, Tribenzylamin, Tricyclohexylamin, Triamylamin, Trihexylamin, N,N-Dimethylanilin, N,N-Diethylanilin, N,N-Diisopropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N-Methylpiperidin, N-Ethylpiperidin, N-Methylpyrrolidin, N-Ethylpyrrolidin, N-Methylpyrrol, N-Methylmorpholin, N-Ethylmorpholin, N-Methylhexamethylenimin, Pyridin, Chinolin, α,β,γ-Picolin, N,N,N′,N′-Tetramethylethylendiamin, N-Ethyldiisopropylamin, N,N-Dimethylcyclohexylamin, Natriumhydrid, Lithiumhydrid, Calciumhydrid. Es können aber auch andere, üblicherweise verwendete basische Stoffe eingesetzt werden.

Als Reaktionsbeschleuniger kommen vorzugsweise Metalliodide und -bromide, beispielsweise Natriumiodid, Kaliumbromid, Calciumiodid; Kronenether; quartäre Ammoniumverbindungen, wie Tetrabutylammoniumiodid oder -bromid; Säuren; oder Kombinationen dieser Stoffe in Frage.

Das erfindungsgemässe Verfahren wird im allgemeinen bei Temperaturen zwischen 0 und 180 °C, vorzugsweise zwischen 40 und 100 °C, während 30 Minuten und 120 Stunden, vorzugsweise 1 Stunde und 60 Stunden, drucklos oder unter Druck, kontinuierlich oder diskontinuierlich, durchgeführt. Bei der Durchführung des erfindungsgemässen Verfahrens setzt man im allgemeinen auf 1 Mol der Verbindung II 0,5 bis 2 Mol der Verbindung III und 0,5 bis 2 Mol Triazol, vorzugsweise 0,9 bis 1,2 Mol Verbindung III und 0,9 bis 1,4 Mol Triazol, sowie 3 bis 6 Mol, vorzugsweise 2,8 bis 3,3 Mol Base und gegebenenfalls 0,01 bis 0,1 Mol des Reaktionsbeschleunigers ein.

In einer bevorzugten Form des erfindungsgemässen Verfahrens vermischt man in beliebiger Reihenfolge die Ausgangsstoffe III und 1,2,4-Triazol mit einer Base und einem organischen Verdünnungsmittel oder Wasser, gibt dann den Ausgangsstoff II und gegebenenfalls einen Reaktionsbeschleuniger zu und führt die Reaktion bei 0 bis 180 °C, vorzugsweise 40 bis 110 °C, während 0,5 und 120, vorzugsweise 1 und 60 Stunden durch. Zur Isolierung der erfindungsgemässen Stoffe giesst man im Falle von Wasser oder mit Wasser mischbaren organischen Verdünnungsmitteln, die gegebenenfalls eingeengte Reaktionsmischung auf Eiswasser und fügt unter Eiskühlung Mineralsäure bis zur stark sauren Reaktion zu. Eventuell

unumgesetztes Triazol wird hierbei abgetrennt, das ausgefallene Reaktionsprodukt wird abgesaugt und zur Entfernung eventuell noch anhaftenden Ausgangsstoffes III nachgewaschen und getrocknet; es bedarf im allgemeinen keiner weiteren Reinigung, kann aber nötigenfalls nach bekannten Methoden wie Umkristallisation, Extraktion oder Chromatographie weiter gereinigt werden. Bei Lösungsmitteln, die mit Wasser nicht mischbar sind, giesst man den Reaktionsansatz in Wasser, trennt die Phasen, extrahiert gegebenenfalls die organische Phase mit wässrigem Alkali oder gesättigter Bicarbonatlösung und isoliert den gewünschten Endstoff durch Ansäuern der wässrig-alkalischen Phase wie oben beschrieben.

Beispiel 1

Zu 81,5 Teilen 2,4-Dichlorphenol und 34,5 Teilen 1,2,4-Triazol in 300 Teilen Ethanol gibt man 90 Teile einer technischen Natriummethylatlösung in Methanol (30 Teile Natriummethylat in 100 Teilen Lösung) und trägt nach kurzem Rühren 109 Teile Dibromessigsäure ein. Man kocht 14 Stunden unter Rückfluss, zieht das Lösungsmittel ab und löst den Rückstand in ca. 1000 Teilen Wasser auf. Man versetzt mit Eis und säuert unter Rühren mit konzentrierter Salzsäure bis pH 1 an, saugt den ausgefallenen Niederschlag ab und wäscht mit Isopropanol nach. Nach dem Trocknen erhält man 121 Teile (84% der Theorie) 2-(2′,4′-Dichlorphenoxy)-2-(1′,2′,4′-triazol-1′-yl)-essigsäure vom Schmelzpunkt 211 bis 213 °C (Zersetzung).

Beispiel 2

Zu 1080 Teilen einer technischen Natriummethylatlösung in Methanol (30 Teile Natriummethylat in 100 Teilen Lösung) gibt man 771 Teile 4-Chlorphenol und 414 Teile 1,2,4-Triazol. Nach kurzem Nachrühren lässt man 774 Teile Dichloressigsäure zulaufen, wobei die Temperatur auf ca. 60 °C ansteigt. Man destilliert nun das Methanol ab und lässt gleichzeitig Isopropanol so nachlaufen, dass das Reaktionsgemisch ohne Schwierigkeiten gerührt werden kann. Hat die Innentemperatur 80 °C erreicht, kocht man 10 Stunden weiter, destilliert dann das Lösungsmittel ab, setzt Eiswasser zu und säuert mit konzentrierter Salzsäure an. Der ausgefallene Niederschlag wird abgesaugt, mit Isopropanol nachgewaschen und getrocknet. Man erhält so 676 Teile (58% der Theorie) 2-(4′-Chlorphenoxy)-2-(1′,2′,4′-triazol-1′-yl)-essigsäure vom Schmelzpunkt 205 bis 207 °C (Zersetzung).

Analog werden beispielsweise folgende Verbindungen der Formel I dargestellt:

Tabelle

| Beispiel Nr. | $R^1$ | $R^2$ | Z | Schmelzpunkt [°C] |
|---|---|---|---|---|
| 3 | 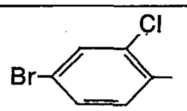 | H | OH | 209–213 (Zers.) |
| 4 | 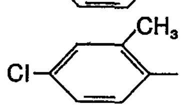 | H | OH | 178–181 (Zers.) |
| 5 | 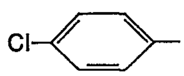 | $CH_3$ | OH | 148–152 |
| 6 | 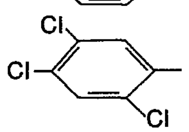 | H | OH | 218–223 (Zers.) |
| 7 | 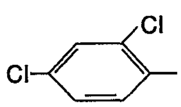 | H | H<br>$ON(C_2H_5)_3$ | 81 |
| 8 | 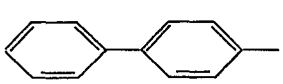 | $CH_3$ | OH | 150–153 |
| 9 | 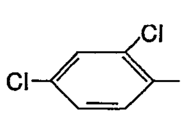 | H | H<br>$ON(C_4H_9)_3$ | |
| 10 | 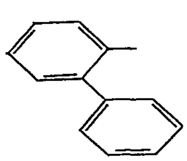 | H | OH | 143–147 |

Tabelle (Fortsetzung)

| Beispiel Nr. | $R^1$ | $R^2$ | Z | Schmelzpunkt [°C] |
|---|---|---|---|---|
| 11 | 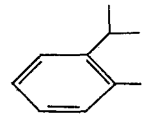 | H | OH | |
| 12 | 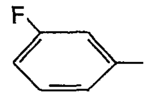 | H | OH | 169–176 (Zers.) |
| 13 | 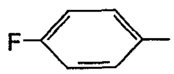 | H | OH | 174–176 |
| 14 | 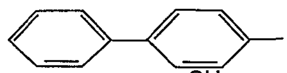 | H | OH | 222–224 |
| 15 | 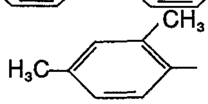 | H | OH | 177–179 (Zers.) |
| 16 | 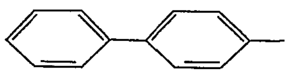 | H | $O\overset{H}{N}(C_2H_5)_3$ | |
| 17 | 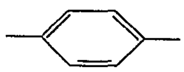 | H | OH | 198–200 |
| 18 | 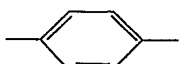 | $CH_3$ | OH | 152–155 |
| 19 | 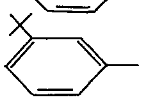 | H | OH | 142–150 |
| 20 | 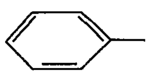 | 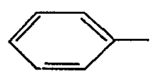 | OH | |
| 21 | 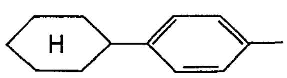 | H | OH | 195–201 |
| 22 | 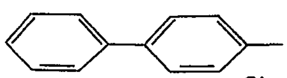 | 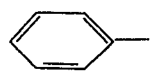 | OH | |
| 23 | 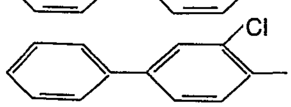 | H | OH | 194–198 (Zers.) |
| 24 | 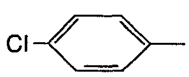 | 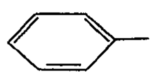 | OH | |
| 25 | 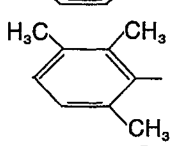 | H | OH | 170–174 (Zers.) |
| 26 | 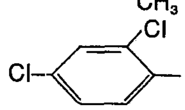 | H | $ONH_3$-tert.-$C_4$-$H_9$ | 156–158 |

Die erfindungsgemässen Verbindungen und ihre Metallkomplexverbindungen besitzen eine hervorragende Wirksamkeit gegen ein breites Spektrum von pflanzenpathogenen Pilzen, insbesondere aus der Klasse der Ascomyceten und Basidiomyceten. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen.

Unter Kulturpflanzen sind in diesem Zusammenhang insbesondere Weizen, Roggen, Gerste, Hafer, Reis, Mais, Baumwolle, Soja, Kaffee, Zuckerrohr, Obst und Zierpflanzen im Gartenbau sowie Gemüse wie Gurken, Bohnen und Kürbisgewächse zu verstehen.

Die neuen Verbindungen sind insbesondere geeignet zur Bekämpfung folgender Pflanzenkrankheiten:

Erysiphe graminis (echter Mehltau) in Getreide,
Erysiphe cichoriacearum (echter Mehltau) an Kürbisgewächsen,
Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie
Helminthosphoriumarten an Getreide,
Ustilago-Arten an Getreide und Zuckerrohr,
Rhynchosporium secale an Getreide,
Venturia inaequalis (Apfelschorf).

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemässen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95 Gew.-% Wirkstoff, vorzugsweise zwischen 0,5 und 90.

Die Aufwandmengen liegen nach Art des gewünschten Effektes zwischen 0,01 und 3, vorzugsweise jedoch zwischen 0,01 und 1 kg Wirkstoff pro Hektar.

Die Mittel bzw. die daraus hergestellten gebrauchsfertigen Zubereitungen, wie Lösungen, Emulsionen, Suspensionen, Pulver, Stäube, Pasten oder Granulate, werden in bekannter Weise angewendet, beispielsweise durch Versprühen, Vernebeln, Verstäuben, Verstreuen, Beizen oder Giessen.

Beispiele für solche Zubereitungen sind:

I. Man vermischt 90 Gewichtsteile der Verbindung des Beispiels 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung des Beispiels 4 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100 000 Gewichtsteilen Wasser erhält man eine wässrige Dispersion, die 0,02 Gew.-% des Wirkstoffs enthält.

III. 20 Gewichtsteile der Verbindung des Beispiels 16 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingiessen und Verteilen der Lösung in 100 000 Gewichtsteilen Wasser erhält man eine wässrige Dispersion, die 0,02 Gew.-% des Wirkstoffs enthält.

IV. 20 Gewichtsteile der Verbindung des Beispiels 19 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gew.-% des Wirkstoffs enthält.

V. 20 Gewichtsteile der Verbindung des Beispiels 21 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge von 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 3 Gewichtsteile der Verbindung des Beispiels 26 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung des Beispiels 43 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig

vermischt. Man erhält auf diese Weise eine Aufbereitung des Wirkstoffs mit guter Haftfähigkeit.

VIII. 40 Gewichtsteile der Verbindung des Beispiels 49 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wässrige Dispersion. Durch Verdünnen mit 100 000 Gewichtsteilen Wasser erhält man eine wässrige Dispersion, die 0,04 Gew.-% Wirkstoff enthält.

IX. 20 Teile der Verbindung des Beispiels 55 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemässen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrösserung des fungiziden Wirkungsspektrums.

Das folgende Beispiel zeigt die biologische Wirkung der neuen Substanzen. Als Vergleichssubstanzen dienten die Verbindungen (2,4-Dichlorphenoxy)-(1,2,4-triazol-1-yl)-essigsäure-amid (X) und (2,4-Dichlorphenoxy)-(1,2,4-triazol-1-yl)-essigsäure-phenylester (Y), vgl. DE-OS 2 720 654, Beispiele 6 und 10.

Beispiel A

Blätter von in Töpfen gewachsenen Weizenkeimlingen der Sorte «Jubilar» werden mit wässrigen Emulsionen aus 80% (Gewichtsprozent) Wirkstoff und 20% Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschliessend im Gewächshaus bei Temperaturen zwischen 20 und 22 °C und 75 bis 80% relativer Luftfeuchtigkeit aufgestellt. Nach 10 Tagen wird das Ausmass der Mehltauentwicklung ermittelt.

| Wirkstoff | Befall der Blätter nach Spritzungen mit ...%iger Wirkstoffbrühe | | |
|---|---|---|---|
| | 0,025 | 0,012 | 0,006 |
| 1 | 0 | 0 | 2 |
| 3 | 0 | 2–3 | 4 |
| 15 | 0 | 0 | 0 |
| 19 | 0 | 0 | 0 |
| 23 | 0 | 0 | 0 |
| X | 4 | 4 | 5 |
| Y | 2 | 3 | 4 |
| Kontrolle (unbehandelt) | 5 | | |

0 = kein Pilzbefall, abgestuft bis 5 = Totalbefall

**Patentansprüche für die Vertragsstaaten: BE, CH, DE, FR, GB, IT, LU, NL, SE**

1. 1,2,4-Triazolderivate der Formel I

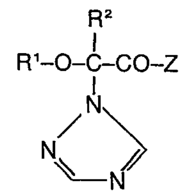

(I)

worin

$R^1$ einen durch 1 bis 3 Halogenatome, $C_{1-4}$-Alkylgruppen, eine Cyclohexyl- oder Phenylgruppe substituierten Phenylrest,

$R^2$ ein Wasserstoffatom, eine $C_{1-6}$-Alkyl- oder Phenylgruppe und

Z eine Hydroxylgruppe oder einen Rest $-\overset{\ominus}{O}\overset{\oplus}{M}$, worin $M^{\oplus}$ für ein Äquivalent eines Metallkations oder ein gegebenenfalls substituiertes Ammoniumion steht,

bedeutet.

2. 2-(2′,4′-Dimethylphenoxy)-2-(1′,2′,4′-triazol-1′-yl)-essigsäure.

3. 2-(3′-tert.-Butylphenoxy)-2-(1′,2′,4′-triazol-1′-yl)-essigsäure.

4. Verfahren zur Herstellung von 1,2,4-Triazolderivaten der Formel I gemäss Anspruch 1, dadurch gekennzeichnet, dass man Verbindungen der Formel II

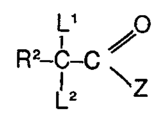

(II),

worin $R^2$ und Z die oben genannten Bedeutungen besitzen und $L^1$ und $L^2$ für nucleophil verdrängbare Abgangsgruppen stehen, mit einer Hydroxyverbindung der Formel III

$$R^1\text{–OH} \qquad \text{(III)},$$

worin $R^1$ die oben genannte Bedeutung hat, oder deren Salzen und mit 1,2,4-Triazol oder dessen Salzen gegebenenfalls in Gegenwart eines Lösungs- oder Verdünnungsmittels und/oder eines Säurefängers und gegebenenfalls in Gegenwart eines Reaktionsbeschleunigers umsetzt.

5. Fungizid, enthaltend mindestens eine Verbindung gemäss Anspruch 1.

6. Fungizid, enthaltend mindestens eine Verbindung gemäss Anspruch 1 und einen festen oder flüssigen Trägerstoff.

7. Verfahren zur Bekämpfung von Fungi, dadurch gekennzeichnet, dass man mindestens eine Verbindung gemäss Anspruch 1 auf diese einwirken lässt.

8. Verfahren zur Herstellung von Fungiziden, dadurch gekennzeichnet, dass man Verbindungen gemäss Anspruch 1 mit festen oder flüssigen Trägerstoffen mischt.

9. Verfahren zur vorbeugenden Bekämpfung von Fungi, dadurch gekennzeichnet, dass man mindestens eine Verbindung gemäss Anspruch 1 auf durch Fungi-Befall bedrohte Flächen, Pflanzen oder Saatgüter einwirken lässt.

10. Verwendung von 1,2,4-Triazolderivaten gemäss Anspruch 1 zur Herstellung von Pflanzenschutzmitteln.

**Patentansprüche für den Vertragsstaat: AT**

1. Verfahren zur Herstellung von 1,2,4-Triazolderivaten der Formel I

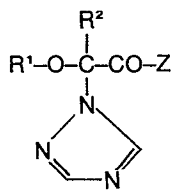

(I)

worin

$R^1$ einen durch 1 bis 3 Halogenatome, $C_{1-4}$-Alkylgruppen, eine Cyclohexyl- oder Phenylgruppe substituierten Phenylrest,

$R^2$ ein Wasserstoffatom, eine $C_{1-6}$-Alkyl- oder Phenylgruppe und

Z eine Hydroxylgruppe oder einen Rest $-O^{\ominus}M^{\oplus}$, worin $M^{\oplus}$ für ein Äquivalent eines Metallkations oder ein gegebenenfalls substituiertes Ammoniumion steht, bedeutet, dadurch gekennzeichnet, dass man Verbindungen der Formel II

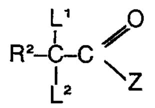

(II),

worin $R^2$ und Z die oben genannten Bedeutungen besitzen und $L^1$ und $L^2$ für nucleophil verdrängbare Abgangsgruppen stehen, mit einer Hydroxyverbindung der Formel III

$R^1$–OH (III),

worin $R^1$ die oben genannte Bedeutung hat, oder deren Salzen und mit 1,2,4-Triazol oder dessen Salzen gegebenenfalls in Gegenwart eines Lösungs- oder Verdünnungsmittels und/oder eines Säurefängers und gegebenenfalls in Gegenwart eines Reaktionsbeschleunigers umsetzt.

2. Fungizid, enthaltend mindestens eine Verbindung gemäss Anspruch 1.

3. Fungizid, enthaltend mindestens eine Verbindung gemäss Anspruch 1 und einen festen oder flüssigen Trägerstoff.

4. Verfahren zur Bekämpfung von Fungi, dadurch gekennzeichnet, dass man mindestens eine Verbindung gemäss Anspruch 1 auf diese einwirken lässt.

5. Verfahren zur Herstellung von Fungiziden, dadurch gekennzeichnet, dass man Verbindungen gemäss Anspruch 1 mit festen oder flüssigen Trägerstoffen mischt.

6. Verfahren zur vorbeugenden Bekämpfung von Fungi, dadurch gekennzeichnet, dass man mindestens eine Verbindung gemäss Anspruch 1 auf durch Fungi-Befall bedrohte Flächen, Pflanzen oder Saatgüter einwirken lässt.

7. Verwendung von 1,2,4-Triazolderivaten gemäss Anspruch 1 zur Herstellung von Pflanzenschutzmitteln.

**Claims for the contracting States: BE, CH, DE, FR, GB, IT, LU, NL, SE**

1. A 1,2,4-triazole derivative of the formula I

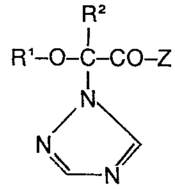

(I)

where

$R^1$ is phenyl substituted by 1 to 3 halogen atoms, alkyl of 1 to 4 carbon atoms, cyclohexyl or phenyl,

$R^2$ is hydrogen, alkyl of 1 to 6 carbon atoms or phenyl, and

Z is hydroxyl or a $-O^{\ominus}M^{\oplus}$ radical, where $M^{\oplus}$ is one equivalent of a metal cation or is an unsubstituted or substituted ammonium ion.

2. 2-(2′,4′-Dimethylphenoxy)-2-(1′,2′,4′-triazol-1′-yl)-acetic acid.

3. 2-(3′-tert.-Butylphenoxy)-2-(1′,2′,4′-triazol-1′-yl)-acetic acid.

4. A process for the preparation of a 1,2,4-triazole derivative of the formula I as claimed in claim 1, wherein a compound of the formula II

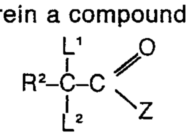

(II),

where $R^2$ and Z have the above meanings and $L^1$ and $L^2$ are nucleophilically displaceable leaving groups, is reacted with a hydroxy compound of the formula III

$R^1$–OH (III)

where $R^1$ has the above meaning, or a salt thereof, and with a 1,2,4-triazole or a salt thereof, in the presence or absence of a solvent or diluent and/or of an acid acceptor and in the presence or absence of a reaction accelerator.

5. A fungicide which contains one or more compounds as claimed in claim 1.

6. A fungicide which contains one or more compounds as claimed in claim 1 and a solid or liquid carrier.

7. A process for combating fungi, wherein one or more compounds as claimed in claim 1 are allowed to act thereon.

8. A process for the preparation of a fungicide, wherein a compound as claimed in claim 1 is mixed with a liquid or solid carrier.

9. A process for the preventive combating of fungi, wherein one or more compounds as claimed in claim 1 are allowed to act on surfaces, plants or seed threatened by fungal attack.

10. Use of a 1,2,4-triazole derivative as claimed in claim 1 for the preparation of crop protection agents.

**Claims for the Contracting State:** AT

1. A process for the preparation of a 1,2,4-triazole derivative of the formula I

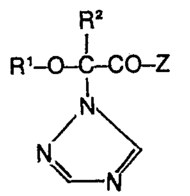

(I)

where

$R^1$ is phenyl substituted by 1 to 3 halogen atoms, alkyl of 1 to 4 carbon atoms, cyclohexyl or phenyl,

$R^2$ is hydrogen, alkyl of 1 to 6 carbon atoms or phenyl, and

Z is hydroxyl or a $-\overset{\ominus\oplus}{OM}$ radical, where $M^{\oplus}$ is one equivalent of a metal cation or is an unsubstituted or substituted ammonium ion, wherein a compound of the formula II

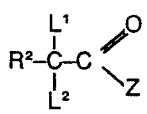

(II),

where $R^2$ and Z have the above meanings and $L^1$ and $L^2$ are nucleophilically displaceable leaving groups, is reacted with a hydroxy compound of the formula III

$R^1$–OH (III)

where $R^1$ has the above meaning, or a salt thereof, and with a 1,2,4-triazole or a salt thereof, in the presence or absence of a solvent or diluent and/or of an acid acceptor and in the presence or absence of a reaction accelerator.

2. A fungicide which contains one or more compounds as claimed in claim 1.

3. A fungicide which contains one or more compounds as claimed in claim 1 and a solid or liquid carrier.

4. A process for combating fungi, wherein one or more compounds as claimed in claim 1 are allowed to act thereon.

5. A process for the preparation of a fungicide, wherein a compound as claimed in claim 1 is mixed with a liquid or solid carrier.

6. A process for the preventive combating of fungi, wherein one or more compounds as claimed in claim 1 are allowed to act on surfaces, plants or seed threatened by fungal attack.

7. Use of a 1,2,4-triazole derivative as claimed in claim 1 for the preparation of crop protection agents.

**Revendications pour les Etats contractants: BE, CH, DE, FR, GB, IT, LU, NL, SE**

1. Dérivés de 1,2,4-triazole de formule I

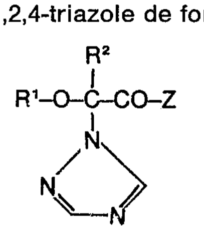

(I)

dans laquelle

$R^1$ représente un reste phényle substitué par 1 à 3 atomes d'halogène, des groupes alkyle en $C_{1-4}$, un groupe cyclohexyle ou phényle,

$R^2$ un atome d'hydrogène, un groupe alkyle en $C_{1-6}$ ou phényle et

Z un groupe hydroxyle ou un reste $-\overset{\ominus\oplus}{OM}$ où $M^{\oplus}$ est mis pour un équivalent d'un cation métal ou un ion ammonium éventuellement substitué.

2. Acide 2-(2′,4′-diméthylphénoxy)-2-(1′,2′,4′-triazol-1′-yl)acétique.

3. Acide 2-(3′-tert.-butylphénoxy)-2-(1′,2′,4′-triazol-1′-yl)acétique.

4. Procédé de préparation de dérivés de 1,2,4-triazole de formule I selon la revendication 1, caractérisé par le fait qu'on fait réagir des composés de formule II

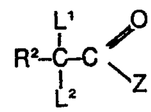

(II),

dans laquelle $R^2$ et Z ont les significations susindiquées et $L^1$ et $L^2$ sont mis pour des groupes de départ nucléophiles déplaçables, avec un composé hydroxy de formule III

$R^1$–OH (III),

où $R^1$ a la signification susindiquée, ou leurs sels, et avec 1,2,4-triazole ou ses sels, éventuellement en présence d'un solvant ou diluant et/ou un fixateur d'acide et éventuellement en présence d'un accélérateur de réaction.

5. Fongicide contenant au moins un composé selon la revendication 1.

6. Fongicide contenant au moins un composé selon la revendication 1 et un support solide ou liquide.

7. Procédé de lutte contre les moisissures, caractérisé par le fait qu'on fait agir sur elles au moins un composé selon la revendication 1.

8. Procédé de préparation de fongicides, caractérisé par le fait qu'on mélange des composés selon la revendication 1 avec des supports solides ou liquides.

9. Procédé de lutte préventive contre les moisissures, caractérisé par le fait qu'on fait agir au moins un composé selon la revendication 1 sur des surfaces, plantes ou semences menacées par l'attaque des moisissures.

10. Utilisation de dérivés de 1,2,4-triazole selon la revendication 1 pour la préparation d'agents de protection des plantes.

**Revendications pour l'Etat contractant: AT**

1. Procédé de préparation de dérivés de 1,2,4-triazole de formule I

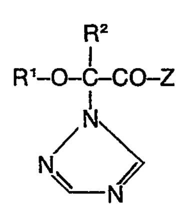

(I)

dans laquelle

$R^1$ représente un reste phényle substitué par 1 à 3 atomes d'halogène, des groupes alkyle en $C_{1-4}$, un groupe cyclohexyle ou phényle,

$R^2$ un atome d'hydrogène, un groupe alkyle en $C_{1-6}$ ou phényle et

Z un groupe hydroxyle ou un reste $-O^{\ominus}M^{\oplus}$ où $M^{\oplus}$ est mis pour un équivalent d'un cation métal ou un ion ammonium éventuellement substitué, caractérisé par le fait qu'on fait réagir des composés de formule II

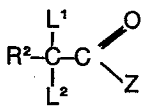

(II),

dans laquelle $R^2$ et Z ont les significations susindiquées et $L^1$ et $L^2$ sont mis pour des groupes de départ nucléophiles déplaçables, avec un composé hydroxy de formule III

$$R^1\text{–OH} \qquad \text{(III)},$$

où $R^1$ a la signification susindiquée, ou leurs sels, et avec 1,2,4-triazole ou ses sels, éventuellement en présence d'un solvant ou diluant et/ou un fixateur d'acide et éventuellement en présence d'un accélérateur de réaction.

2. Fongicide contenant au moins un composé selon la revendication 1.

3. Fongicide contenant au moins un composé selon la revendication 1 et un support solide ou liquide.

4. Procédé de lutte contre les moisissures, caractérisé par le fait qu'on fait agir sur elles au moins un composé selon la revendication 1.

5. Procédé de préparation de fongicides, caractérisé par le fait qu'on mélange des composés selon la revendication 1 avec des supports solides ou liquides.

6. Procédé de lutte préventive contre les moisissures, caractérisé par le fait qu'on fait agir au moins un composé selon la revendication 1 sur des surfaces, plantes ou semences menacées par l'attaque des moisissures.

7. Utilisation de dérivés de 1,2,4-triazole selon la revendication 1 pour la préparation d'agents de protection des plantes.